(19) Europäisches Patentamt
European Patent Office
Office européen des brevets

(11) Publication number: 0 076 628 A2

(12) # EUROPEAN PATENT APPLICATION

(21) Application number: 82305137.0

(22) Date of filing: 29.09.82

(51) Int. Cl.³: C 07 D 249/08
C 07 D 233/58, C 07 D 233/60
A 01 N 43/64, A 01 N 43/50

(30) Priority: 01.10.81 US 307414

(43) Date of publication of application: 13.04.83 Bulletin 83/15

(84) Designated Contracting States: AT BE CH DE FR GB IT LI NL SE

(71) Applicant: Rohm and Haas Company
Independence Mall West
Philadelphia, Pennsylvania 19105(US)

(72) Inventor: Miller, George Allen
1812 Fort Washington Avenue
Maple Glen Pennsylvania 19002(US)

(72) Inventor: Chan, Hak-Foon
524 Matterhorn Drive
Walnut Creek California 94598(US)

(74) Representative: Wood, Peter Worsley Spencer et al,
Rohm and Haas Company Patent Department
Chesterfield House Bloomsbury Way
London, WC1A 2TP(GB)

(54) **Novel substituted imidazoles and triazoles, fungicidal compositions containing them and the use of the novel compounds for combating fungi.**

(57) Novel compounds are provided which exhibit fungicidal activity against phytopathogenic fungi. The novel compounds are of Formula I below:

$$ZR^1C = CQR^2 \qquad (I)$$

wherein:

Z is phenyl or naphthyl each being optionally substituted with up to three of the same or different substituents selected from halogen, nitro, trihalomethyl, cyano, ($C_1$-$C_6$)alkyl, ($C_1$-$C_6$)alkoxy, ($C_1$-$C_6$)alkylthio, ($C_1$-$C_6$)alkylsulfinyl, ($C_1$-$C_6$) alkylsulfonyl and ($C_2$-$C_6$)alkenyl;

$R^1$ is hydrogen, ($C_1$-$C_{12}$)alkyl, ($C_3$-$C_8$)cycloalkyl, ($C_2$-$C_8$)alkenyl, ($C_5$-$C_8$)cycloalkenyl, ($C_2$-$C_8$)alkynyl, phenyl, naphthyl each of the last groups being optionally substituted with up to three of the same or different substituents selected from halogen, nitro, trihalomethyl, cyano, ($C_1$-$C_6$)alkyl, ($C_2$-$C_6$)alkenyl, ($C_2$-$C_6$)-alkynyl, ($C_1$-$C_6$)alkoxy, ($C_1$-$C_6$)alkylsulfinyl and ($C_1$-$C_6$)alkylsulfonyl;

$R^2$ is hydrogen, phenyl optionally substituted with up to three of the same or different substituents selected from nitro, trihalomethyl, cyano, ($C_1$-$C_6$)alkyl, ($C_2$-$C_6$)alkenyl, ($C_2$-$C_6$)alkynyl, ($C_1$-$C_6$)alkoxy, ($C_1$-$C_6$)alkylsulfinyl and ($C_1$-$C_6$)-alkylsulfonyl; and

Q is 1-imidazolyl, or 1- or 4-(1,2,4-triazolyl).

EP 0 076 628 A2

# NOVEL SUBSTITUTED IMIDAZOLES AND TRIAZOLES, FUNGICIDAL COMPOSITIONS CONTAINING THEM AND THE USE OF THE NOVEL COMPOUNDS FOR COMBATING FUNGI

This invention concerns the provision of new fungicidal imidazoles and triazoles, fungicidal compositions containing them and methods of combating fungi.

Certain substituted imidazole and triazoles are known as fungicides, see for example UK Patent Specification Nos. 1,530,172 and 1,601,423 which disclose, inter alia, fungicidal compounds of the formula:

$$Z'-A-Q'$$

where Z' is optionally substituted phenyl or naphthyl, A is a straight chain alkylene group substituted by various substituents such as alkyl, phenyl or substituted phenyl and Q' is triazolyl or imidazolyl. Metal salt complexes and acid addition salts of compounds of the above formula are also disclosed.

The fungicidal effectiveness of a substituted imidazole or triazole cannot be predicted from its structure only and often quite closely related compounds will have quite different fungi control abilities.

An ideal fungicide should give selective fungi control, over the full growing season, with a single administration at low doses; it should also have a wide fungicidal spectrum. At the same time, the fungicide should be substantially non-phytotoxic to the crops to which it is applied and should not leave toxic residues on crop plants or the soil. The known fungicidal substituted imidazoles and triazoles fall short of these ideals and there is need for new fungicides having different or improved fungicidal activity.

The novel substituted imidazoles and triazoles provided

by the invention are somewhat similar in structure to the above-described prior art compounds but are characterized in that the groups Z' and Q' of the prior art compound are linked with an optionally substituted ethylenic linkage.
More precisely the new compounds of the invention are those of the formula:

$$ZR^1C{=}CQR^2 \qquad (I)$$

wherein:

Z is phenyl or naphthyl each being optionally substituted with up to three of the same or different substituents selected from halogen, nitro, trihalomethyl, cyano, $(C_1\text{-}C_6)$alkyl, $(C_1\text{-}C_6)$alkoxy, $(C_1\text{-}C_6)$alkylthio, $(C_1\text{-}C_6)$alkylsulfinyl, $(C_1\text{-}C_6)$alkylsulfonyl and $(C_2\text{-}C_6)$-alkenyl;

$R^1$ is hydrogen, $(C_1\text{-}C_{12})$alkyl, $(C_3\text{-}C_8)$cycloalkyl, $(C_2\text{-}C_8)$alkenyl, $(C_5\text{-}C_8)$cycloakenyl, $(C_2\text{-}C_8)$-alkynyl, phenyl, naphthyl each of the last two groups being optionally substituted with up to three of the same or different substituents selected from halogen, nitro, trihalomethyl, cyano, $(C_1\text{-}C_6)$alkyl, $(C_2\text{-}C_6)$alkenyl, $(C_2\text{-}C_6)$-alkynyl, $(C_1\text{-}C_6)$alkoxy, $(C_1\text{-}C_6)$alkylsulfinyl and $(C_1\text{-}C_6)$alkylsulfonyl;

$R^2$ is hydrogen, phenyl optionally substituted with up to three of the same or different substituents selected from nitro, trihalomethyl, cyano, $(C_1\text{-}C_6)$alkyl, $(C_2\text{-}C_6)$alkenyl, $(C_2\text{-}C_6)$alkynyl, $(C_1\text{-}C_6)$alkoxy, $(C_1\text{-}C_6)$alkylsulfinyl and $(C_1\text{-}C_6)$-alkylsulfonyl; and

Q is 1-imidazolyl, or 1- or 4-(1,2,4-triazolyl).

Of course, the invention also embraces enantiomorphs

and geometric isomers of compounds of Formula I above. Also included within the scope of the invention are acid addition salts and metal salt complexes of compounds of Formula I eg metal salt complexes containing at least one metal cation selected from magnesium, manganese, copper, nickel, zinc, iron, cobalt, calcium, mercury, chromium, lead and barium.

One class of compounds of Formula I are those (including the acid addition salts and metal salt complexes) wherein Z is phenyl optionally substituted with up to two substituents selected from chlorine, bromine, $(C_1-C_4)$alkyl, nitro, trifluoromethyl, cyano and $(C_1-C_4)$alkoxy; $R^1$ is hydrogen, $(C_1-C_8)$alkyl, phenyl optionally substituted with up to two of the same or different substituents selected from chlorine, bromine, nitro, trifluoromethyl, cyano, $(C_1-C_4)$alkyl and $(C_1-C_4)$alkoxy; and Q is 1-imidazolyl, 1- or 4-(1,2,4-triazolyl).

Another class of compounds of Formula I are those (including the acid additon salts and metal salt complexes) wherein Z is phenyl optionally substituted with up to two substituents selected from halo and $(C_1-C_4)$alkyl, $R^1$ is hydrogen, $(C_1-C_6)$alkyl or phenyl optionally substituted with up to two of the same or different substituents selected from $(C_1-C_4)$alkyl or halogen, $R^2$ is hydrogen or phenyl substituted with up to two of the same or different halogen atoms and Q is 1-imidazolyl or 1-(1,2,4-triazolyl).

In yet another class of compounds of Formula I are those (including the acid addition salts and metal salt complexes) wherein Z is phenyl or phenyl substituted with up to two substituents selected from chlorine, bromine and $(C_1-C_4)$alkyl; $R^1$ is hydrogen, $(C_1-C_6)$alkyl,

phenyl, or phenyl substituted with up to two substituents selected from chloro, bromo and $(C_1-C_4)$-alkyl; $R^2$ is hydrogen, phenyl, or phenyl substituted with up to two substituents selected from chlorine, bromine and $(C_1-C_4)$alkyl; and Q is 1-imidazolyl or 1-(1,2,4-triazolyl). In this class of compounds preferred are those wherein Z is phenyl, 4-chlorophenyl, 2-chlorophenyl or 2,4-dichlorophenyl; $R^1$ is hydrogen, $(C_1-C_4)$alkyl, phenyl, 2-chlorophenyl, or 4-chlorophenyl and $R^2$ is hydrogen, phenyl, or 2-chlorophenyl.

Examples of Z, $R^1$ and $R^2$ moieties in Formula I are phenyl, 2-chlorophenyl, 4-chlorophenyl, 2,4-dichlorophenyl, 2,4-dibromophenyl, 3,5-difluorophenyl, 3,4-dichlorophenyl, 2-chloro-4-iodophenyl, 3-chloro-4-nitrophenyl, 2,4-dinitrophenyl, 3,4,5-trimethylphenyl, 2-nitro-4-methoxyphenyl, 4-ethylphenyl, 2,4-dimethoxyphenyl, 4-trifluoromethylphenyl, 2-nitro-4-trifluoromethylphenyl, 3,5-dimethylthiophenyl, 2-cyano-5-methylphenyl, 2,4-dimethylsulfinylphenyl, 2,4-dimethylsulfonylphenyl and 2-iodo-4-methylphenyl. In addition, typical Z and $R^1$ moieties include, for example, naphthyl, 2-chloronaphthyl, 2-nitronaphthyl and 2,4-diiodonaphthyl.

The term "alkyl" as used in this specification includes of course both straight and branched chain alkyl groups. Typical alkyl groups are methyl, ethyl, propyl, isopropyl, n-butyl, sec-butyl, iso-butyl, tert-butyl, pentyl, neopentyl, iso-pentyl, hexyl, heptyl, iso-octyl, nonyl, decyl, iso-decyl, undecyl and dodecyl.

The acids which can be used in making the acid addition salts of the present invention include hydrochloric, hydrobromic, nitric, sulfinic, phosphoric, hydroiodic, hydrofluoric, perchloric, p-toluene-sulfonic, methanesulfonic, acetic, citric, tartaric, malic, maleic, oxalic, fumaric, phthalic and the like.

A sub-class of metal salt complexes may be expressed by the formula

$$Z - \underset{R^1}{\overset{|}{C}} = \underset{R^2}{\overset{|}{C}} - Q \quad \cdot M_aX_b \qquad \text{(II)}$$

wherein Z, $R^1$, $R^2$ and Q are as defined in Formula I above, a and b are integers, M is a cation selected from Group IIA, IB, IIB, VIB, or VII of the Periodic Table, and X is an anionic counterion selected in such a manner that the sum of the valence charges of the a M cations and the b X anions equals zero. The Periodic Table referred to above is that appearing on the front pages of the Merck Index, 9th Edition.

Typical cations in the metal salt complexes are at least one of magnesium, calcium, manganese, copper, nickel, zinc, iron, cobalt, tin, cadmium, mercury, chromium, lead and barium.

Typical anions in the metal complexes are chloride, bromide, iodide, fluoride, sulfate, bisulfate, perchlorate, nitrate, nitrite, phosphate, carbonate, bicarbonate, acetate, citrate, oxalate, tartarate, malate, maleate, fumarate, p-toluenesulfonate, methanesulfonate, mono- or di-$(C_1-C_4)$alkyldithiocarbamate and $(C_1-C_4)$alkylenebisdithiocarbamate.

Typical compounds of Formula I include:

1-[1,2-di-(2-chlorophenyl)ethenyl] imidazole

1-[2-(2,4-dichlorophenyl)hex-1-enyl] imidazole

1-[2,2-bis(4-chlorophenyl)ethenyl]-1,2,4-triazole

1-[2,2-bis(phenyl)ethenyl] imidazole hydrochloride

1-[2,2-bis(4-chlorophenyl)ethenyl] imidazole hydrochloride

1-[2,2-bis(4-chlorophenyl)ethenyl] imidazole

1-[2,2-bis(phenyl)ethenyl] imidazole
1-[2,2-bis(4-chlorophenyl)ethenyl] imidazole
1-[2-(4-chlorophenyl)-2-(2-chlorophenyl)ethenyl]-1,2,4-triazole
1-[2-(4-chlorophenyl)-2-(4-ethylphenyl)ethenyl]-1,2,4-triazole hydrochloride
1-[2-(4-chlorophenyl)-2-(4-ethylphenyl)ethenyl]-1,2,4-triazole
1-[2,2-bis(4-chlorophenyl)ethenyl]-1,2,4-triazole hydrochloride
1-[2-(4-chlorophenyl)-2-(2-chlorophenylethenyl]-1,2,4-triazole
1-[2-(2,4-dichlorophenyl)-hex-1-enyl]-1,2,4-triazole
1-[2,2-bis(4-ethylphenyl)ethenyl]-1,2,4-triazole
1- [2,2-biz(4-ethylphenyl)ethenyl]-1,2,4-triazole hydrochloride

The phenylethenyl imidazoles and triazoles of the present invention can be prepared by standard elimination reactions from the corresponding saturated compounds. A preferred method for preparing the imidazoles and triazoles of the invention is as follows:

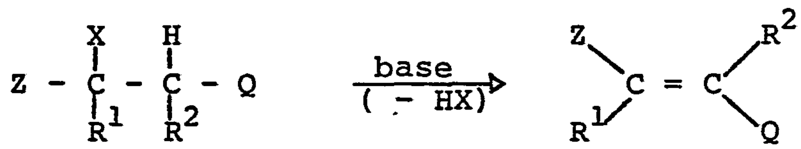

(Eq. 1)

(A) (B)

where Z, $R^1$, $R^2$ and Q are defined as in Formula I above and X is halogen, $(C_1-C_8)$alkylsulfonyl, phenylsulfonyl optionally substituted on the phenyl ring with up to three substituents selected from halogen and $(C_1-C_8)$ alkyl or other appropriate leaving group. The base employed can be an organic base such as pyridine,

$(C_1-C_{12})$alkyl amine, di-$(C_1-C_{10})$alkylamine, tri-$(C_1-C_{10})$alkylamine or an inorganic base such as an alkali or alkaline earth metal hydroxides, alkali metal carbonates, or a $(C_1-C_{12})$alkoxide of an alkali metal. The elimination reaction can be performed without solvent or in an organic solvent such as dimethylsulfoxide, dimethyl formamide, benzene, toluene or xylene.

The intermediate halide (A) in Equation 1 above can be prepared from the corresponding hydroxide by treatment of the hydroxide with thionyl chloride, methane sulfinyl chloride or phosphorus tribromide as shown below:

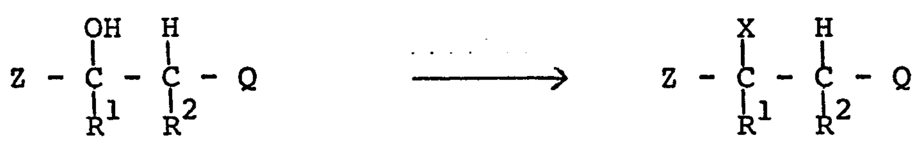

(Eq. 2)

where Z, $R^1$, $R^2$, Q and X are defined above.

The preparation of certain hydroxyphenethyl imidazoles and triazoles useful as starting materials in Equation 2 is described in the published version of Japanese Patent Application No. 78/90019.

For the preparation of the diphenyl ethenyl imidazoles and triazoles, two potential leaving groups are required, one for the generation of the carbon-carbon double bond and the other for the attachment of the azo ring via a substitution reaction. For example, reaction of 2,2-bis(p-chlorophenyl)-1,1-dichloroethane, with 50% by weight aqueous sodium hydroxide solution in dimethyl sulfoxide (DMSO) provides vinyl chloride in quantitative yield. Without further purification, vinyl chloride can be used to react directly with imidazole or sodium imidazole to furnish the final product, 2,2-bis(p-chlorophenyl)ethenyl imidazole (Equation 3).

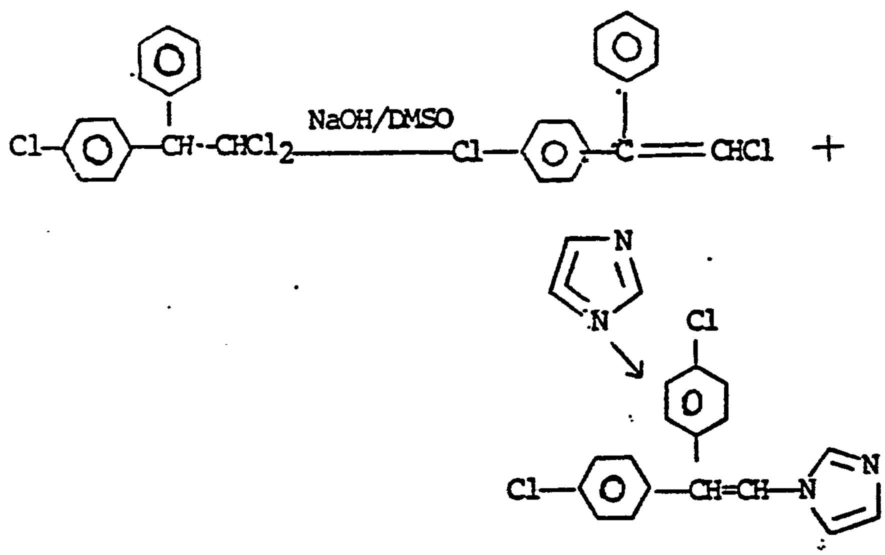


(Eq. 3)

The starting material in this reaction can be produced by reacting chlorobenzene with dichloracetaldehyde under the Friedel-Crafts conditions (Equation 4).

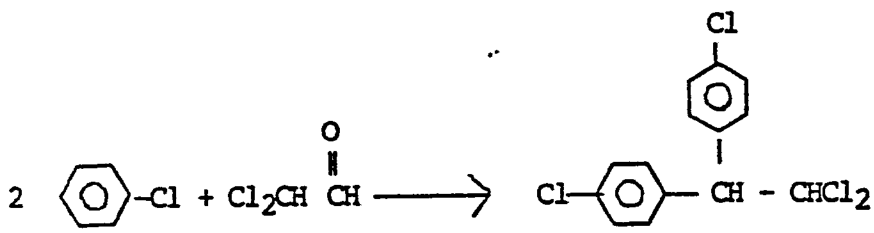

(Eq. 4)

The acid addition salts of the ethylenic imidazoles and triazoles of this invention can be prepared by standard techniques well-known in the art. For example, a compound of Formula (I) can be dissolved in an appropriate solvent such as diethyl ether, tetrahydrofuran, ethanol, methanol or combinations thereof, and treated with an equivalent or excess amount of a mineral or organic acid which may or may not be dissolved in an appropriate solvent. The mixture is then either cooled or evaporated to give the salt, which can either be used as such or recrystallized from an appropriate solvent or combination of appropriate solvents.

The metal salt complexes of the ethylenic imidazoles and triazoles can be prepared by adding dropwise, with stirring, a stoichiometric amount of a metal salt dissolved in an appropriate solvent or combination of solvents to a solution of the ethylenic imidazole or triazole of Formula (I) dissolved in a similarly appropriate solvent or combination of solvents. The reaction mixture is briefly stirred and the solvent is removed under reduced pressure to give the metal salt complex of the respective ethylenic imidazole or triazole of Formula (II).

The metal salt complexes can also be prepared by mixing stoichiometric or excess amounts of the metal salt and an ethylenic imidazole or triazole of Formula (I) in the desired amount of solvent containing the appropriate adjuvants just prior to spraying the plants. Adjuvants that may be included in this "in-situ" preparation may be detergents, emulsifiers, wetting agents, spreading agents, dispersing agents, stickers, adhesives, and the like which are used in agricultural applications.

Solvents that can be utilized in these procedures include any polar solvent, e.g., water, methanol, ethanol, isopropanol or ethylene glycol and any aprotic polar solvent, e.g., dimethylsulfoxide, acetonitrile, dimethylformamide, nitromethane or acetone.

Any metal containing fungicides can be used in place of the metal salt. Typical metal containing fungicides that can be utilized in these procedures are: a) dithiocarbamates and derivatives such as: ferric dimethyldithiocarbamate (ferbam), zinc dimethyldithiocarbamate (ziram), manganese ethylenebisdithiocarbamate (maneb) and its coordination product with zinc ion (mancozeb), zinc ethylenebisdithiocarbamate (zineb); b) copper-based fungicides such as cuprous oxide, copper oxychloride, copper naphthenate, and Bordeaux mixture; and c) miscellaneous fungicides such as: phenylmercuric acetate, N-ethylmercuri-1,2,3,6-tetrahydro-3,6-endomethano-3,4,5,6,7,7-hexachlorophthalimide, phenylmercuri monoethanolammonium lactate, nickel-containing compounds and calcium cyanamide.

Some of the compounds of this invention possess a asymmetric carbon atom (i.e., in $R_1$ or $R_2$ of Formula I) and thus exist as racemic mixtures. The d and l enantiomorphs in these racemic mixtures can be separated via standard techniques such as fractional crystallization with d-tartaric acid, l-tartaric acid or l-quinic acid followed by basification and extraction of the d or l enantiomorph free base.

The following examples are provided merely to illustrate the method of preparation of the compounds of the present invention. The temperatures expressed in these examples are in degrees centigrade. Percentages are on a weight basis unless stated otherwise.

## Example 1

### 1-[1,2-Di-(2-chlorophenyl)ethenyl] imidazole

A. Preparation of 1-[2-chloro-1,2-di-(2-chlorophenyl) ethyl] imidazole

A solution of 6 g (0.05 mole) of thionyl chloride dissolved in 20 ml of benzene is added dropwise to a solution containing 11 g (0.033 mole) of 1-[2-hydroxy-1,2-di-(2-chlorophenyl)ethyl] imidazole and 150 ml of benzene at room temperature. The reaction mixture is heated to reflux for 3 hours and is then made basic with 10% ammonium hydroxide solution. The organic product is extracted with ether and the combined ether extracts are washed with water, saturated sodium chloride solution, and dried over magnesium sulfate. Solvent is evaporated to give 11 g of a brown oil. This material is further purified by converting to its hydrochloride salt and back neutralized to the free base to give 8.2 g of the expected product.

nmr ($CDCl_3$): 6.2 - 6.6 (two doublets centered around 6.4, 2H), 6.7 - 8.0 (complex multiplets, 11H)

B. Preparation of 1-[1,2-Di-(2-chlorophenyl)ethenyl] imidazole

To a solution containing 3 g of 1-[2-chloro-1,2-di-(2-chlorophenyl)ethyl] imidazole in 20 ml of dimethyl sulfoxide is added 10 ml of 50% sodium hydroxide solution dropwise. The resulting reaction mixture is heated at 60° for 2 hours and poured into 300 ml of water and extracted with ether. The combined ether extracts are washed with water, saturated sodium chloride solution and dried over magnesium. Solvent is evaporated to give 2.5 g of a yellow oil.

Example 2

1-[2-(2,4-Dichlorophenyl)hex-1-enyl) imidazole

A. Preparation of 1-[2-Chloro-2-(2,4-dichlorophenyl)hexyl] imidazole

To a solution of 15 g (0.05 mole) of 1-[2-hydroxy-2-(2,4-dichlorophenyl)hexyl] imidazole (in 100 ml of chloroform is added 6 g (0.05 mole) of thionyl chloride dropwise. After the addition, the reaction is heated to reflux for 8 hours. The reaction mixture is then poured into water and extracted with chloroform. The combined chloroform extracts are washed with 10% ammonium hydroxide solution, water, saturated sodium chloride solution and dried over magnesium sulfate. Solvent is evaporated to give the crude product which can be further purified by converting to its nitric acid salt and back neutralized to its free base to give 12 g of an oily product.

B. Preparation of 1-[2-(2,4-Dichlorophenyl)hex-1-enyl] imidazole

To a solution of 10 g of 1-[2-chloro-2-(2,4-dichlorophenyl) hexyl] imidazole in 100 ml of dimethyl sulfoxide is added 25 ml of 50% sodium hydroxide solution dropwise at room temperature. The reaction mixture is heated at 60° for 2 hours. It is then poured into 300 ml of ice water and extracted with ether. The combined ether extracts are washed with saturated sodium chloride solution and dried over magnesium sulfate. Drying agent is filered and to the filtrate is added concentrated nitric acid dropwise until it is strongly acidic. An oily material settles and is triturated with a 50-50/ether-hexane (by volume) mixture three times and then back neutralized to its free base with 10% ammonium hydroxide solution. The free base is extracted with ether and the combined ether extracts are washed with water and saturated sodium chloride solution and dried over magnesium sulfate. Solvent is evaporated to give 6 g of a brown oily product.

nmr ($CDCl_3$): 0.6 - 1.5 (m, 7H), 2.6 (t, 2H), 6.6 (s, 1H), 7.0 - 7.8 (m, 6H)

Example 11

1-[2,2-Bis-(p-chlorophenyl)ethenyl]-1,2,4-triazole

A. Preparation of 2,2-Bis(p-chlorophenyl)-1-chloroethylene

Into a 4-necked 2-liter flask are placed 500 g (1.56 mole) of 2,2-bis(p-chlorophenyl)-1,1-dichloroethane and 500 ml of dimethyl sulfoxide. A solution of 188 ml (2.34 mole) of 50% sodium hydroxide is added dropwise. The reaction mixture exotherms to 63° during the addition. After the addition, the reaction mixture is stirred for one hour and poured into ice-water. It is extracted with ether. The combined ether extracts are washed with water, saturated sodium chloride solution, and dried over sodium sulfate. Solvent is evaporated to give 311 g of a waxy solid. A portion of the crude product is recrystalized from hexane-ether to give a white solid, mp 65-7°.

B. 1-[2,2-Bis(p-chlorophenyl)ethenyl]-1,2,4-triazole

Into a 500 ml round bottom flask are charged 12 g (0.17 mole) of 1H-1,2,4-triazole, 11 g (0.17 mole) of 86% powdered potassium hydroxide and 200 ml of dimethyl sulfoxide. The mixture is heated to 140°C and 100 ml of dimethyl sulfoxide are distilled under reduced pressure followed by dropwise addition of 40 g (0.14) of 2,2-bis (p-chlorophenyl)-1-chloroethylene dissolved in 50 ml of dimethyl sulfoxide. The reaction mixture is heated at 140°C for one hour, poured into water, and extracted with ether. The combined ether extracts are washed with water, saturated sodium chloride solution, and dried further purified by trituration with n-hexane to give 25 g of the expected product, mp 126-8°.

nmr(DMSO) 7.0-7.6 (M, 8H0, 7.7 (S, 1H), 8.0 (S, 1H),
8.2 (S, 1H)

In TABLE I, the structures of the above-identified representative compounds of this invention and of additional representative compounds of this invention prepared by the methods described above are set forth. In TABLE II, the melting points and elemental analyses of Examples 1-16 are provided.

Table I

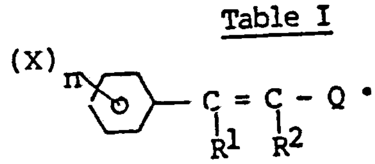 (acid addition salt)

| Example | $(X)_n$ | $R^1$ | $R^2$ | Q | Acid Addition salt |
|---|---|---|---|---|---|
| 1 | 2-Cl | H | 2-Cl $C_6H_4$ | Imidazole | — |
| 2 | 2,4-$Cl_2$ | n-$C_4H_9$ | H | " | |
| 3 | 4-Cl | 4-Cl$C_6H_4$ | " | " | HCl |
| 4 | H | $C_6H_5$ | " | " | HCl |
| 5 | H | " | " | " | — |
| 6 | 4-Cl | 4-Cl$C_6H_4$ | " | " | — |
| 7 | " | " | " | " | $HNO_3$ |
| 8 | " | 2-Cl$C_6H_4$ | " | " | — |
| 9 | 4-$C_2H_5$ | 4-($C_2H_5$)$C_6H_4$ | " | " | HCl |
| 10 | " | " | " | " | — |
| 11 | 4-Cl | 4-Cl$C_6H_4$ | " | 1-(1,2,4-triazolyl) | — |
| 12 | " | " | " | " | HCl |
| 13 | " | 2-Cl$C_6H_4$ | " | " | — |
| 14 | 2,4-$Cl_2$ | n-$C_4H_9$ | " | " | — |
| 15 | 4-$C_2H_5$ | 4-($C_2H_5$)$C_6H_4$ | " | " | HCl |
| 16 | " | " | " | " | — |

TABLE II

Elemental Analysis

| Example | Formula | MP (°C) | C | H | Cl | N |
|---|---|---|---|---|---|---|
| 1 | $C_{17}H_{12}Cl_2N_2$ | Oil | 64.78(64.79) | 3.84(3.80) | 22.50(22.25) | 8.89(8.49) |
| 2 | $C_{15}H_{16}Cl_2N_2$ | Oil | 61.03(60.88) | 5.46(5.50) | 24.02(24.53) | 9.49(9.27) |
| 3 | $C_{17}H_{12}Cl_2 \cdot HCl$ | 240 | 58.06(58.05) | 3.73(3.73) | 30.25(30.41) | 7.97(8.36) |
| 4 | $C_{17}H_{14}N_2 \cdot HCl$ | 200-205 | 68.06(68.56) | 7.42(7.29) | 13.39(13.49) | 10.49(10.66) |
| 5 | $C_{17}H_{14}N_2$ | Oil | 83.25(82.86) | 5.83(5.73) | | 11.24(11.42) |
| 6 | $C_{17}H_{12}Cl_2N_2$ | 93-6 | 64.78(62.94) | 3.84(3.97) | 22.50(21.49) | 8.89(8.58) |
| 7 | $C_{17}H_{12}Cl_2N_2 \cdot HNO_3$ | 147-9 | 53.99(53.58) | 3.46(3.62) | 18.75(18.34) | 11.11(11.58) |
| 8 | $C_{17}H_{12}Cl_2N_2$ | 66-70 | 64.78(65.64) | 3.84(3.97) | 22.50(21.89) | 8.89(8.91) |
| 9 | $C_{21}H_{22}N_2 \cdot HCl$ | 210 | 74.13(74.43) | 6.87(6.84) | 10.61(10.46) | 8.36(8.27) |
| 10 | $C_{21}H_{22}N_2$ | Oil | 81.61(83.40) | 7.29(7.33) | | 8.90(9.27) |
| 11 | $C_{16}H_{11}Cl_2N_3$ | 126-8 | 60.78(60.72) | 3.51(3.45) | 22.42(22.20) | 13.29(13.24) |
| 12 | $C_{16}H_{11}Cl_2N_3 \cdot HCl$ | 185-190 | 54.49(54.17) | 3.43(3.37) | 30.16(30.44) | 11.92(11.68) |
| 13 | $C_{16}H_{11}Cl_2N_3$ | Oil | 60.78(60.27) | 3.51(3.52) | 22.42(22.37) | 13.29(12.45) |
| 14 | $C_{14}H_{15}Cl_2N_3$ | Oil | 56.77(54.65) | 5.10(5.70) | 23.94(21.98) | 14.19(10.86) |
| 15 | $C_{20}H_{21}N_3 \cdot HCl$ | 166-169 | 70.15(70.68) | 6.66(6.52) | 10.39(10.44) | 12.02(12.37) |
| 16 | $C_{20}H_{21}N_3$ | Oil | 78.46(79.17) | 7.06(6.98) | | 13.50(13.85) |

The ethylenic imidazoles and triazoles, acid addition salts and metal salt complexes are broad-spectrum fungicides which possess a high degree of activity against assorted phytopathogenic fungi. The fungicides can be used in concentrations of from about 50 to about 2000 ppm in liquid carrier in controlling barley net blotch (Helminthosporium teres) on barley plants, chocolate spot (Botrytis fabae) on faba beans, bean powdery mildew (Erysiphe polygoni) on bean plants, grape downy mildew (Plasmopora viticola) on grape seedlings, peanut cercospora (Cercospora arachidicola) on peanuts, rice blast (Piricularia oryzae) on rice plants, tomato late blight (Phytophthora infestans) on tomato seedlings and wheat stem rust (Puccinia graminis f. sp. tritici race 15B-2) on wheat seedlings.

In evaluating the fungicides, a fungicidal evaluation is carried out using the compounds at 300 ppm in liquid carrier and spraying the plants to run off using carrier volume of about 1400 l/ha 150 U.S. gall/acre).

The general procedure is to take potted plants in proper condition of growth for susceptibility to the fungal disease to be evaluated, apply the liquid fungicide composition to run off, and allow them to dry. The plants are then inoculated with fungal spores and then allowed to incubate until the disease has developed and the percent control can be read or estimated.

The following test methods are employed in evaluating the fungicidal activity of the compounds concerned in this invention.

EXAMPLE A - Barley Net Blotch (Helminthosporium teres)

Barley plants (var. Besbar or Wong) are trimmed to a height approximately 6.35 cm, 24 hours prior to chemical

application. This procedure provides plants of a uniform height and permits rapid inoculation of treated plants.

*Helminthosporium teres* is cultured on potato-dextrose agar (PDA) slants for 14 days at ambient temperature and low light intensity. Spores are harvested by adding deionised water to the PDA slants and scraping the agar surface with a glass rod with a rubber tip. The spore suspension is filtered through cheesecloth to remove mycelial and agar fragments and then adjusted to a concentration of 15-20,000 spores/ml. One drop (0.5 ml) of polyoxyethylene(20)sorbitan mono-oleate (Tween 80) is added to 100 ml inoculum to provide a more even spore distribution on the surface of the barley leaves.

The barley plants are inoculated by spraying the foliage of the plants with a hand sprayer until small droplets of the inoculum are observed on the leaves. Inoculated plants are incubated in a humid environment at 24-27°C. for 24 hours prior to being placed in the greenhouse at 21-24°C.

Treatment comparisons are made 6-7 days after inoculation. Typical barley net blotch symptoms initially appear as irregular sunken watersoaked areas which become necrotic as the lesions enlarge. Certain of the ethylenic imidazoles and triazoles of this invention demonstrate complete control over *Helminthosporium teres* at an application rate of 300 ppm.

## EXAMPLE B - Chocolate Spot of Broad Beans (*Botrytis fabae*) (BOT)

Broad bean (*Vicia faba*) are trimmed to a height of approximately 11 cm, 24 hours prior to chemical application. This procedure provides plants of a uniform height and permits rapid and uniform inoculation of treated plants.

Botrytis fabae is cultured on oatmeal agar (OA) slants for 21 days at ambient temperature and low light intensity. Spores are harvested by adding deionized water to the OA slants and scraping the agar surface with a rubber tipped glass rod. The spore suspension is filtered through cheesecloth to remove mycelial and agar fragments and then adjusted to a concentration of 175 - 200,000 spores per ml with an inoculation medium. The inoculation medium (20 gms glucose, 1 gm ammonium phosphate, 2 gm potassium nitrate, 10 mgm ascorbic acid, 1500 ml deionized water and 500 ml apple juice) is to provide improved spore germination on the surface of the broad bean leaves and stems.

Broad bean plants are inoculated by spraying the foliage with the fungicide group's overhead mechanical sprayer. Inoculated plants are incubated in a humid environment at 24-27°C for 66 hours.

Treatment comparisons are made 66 - 68 hours after inoculation. Typical broad bean chocolate leaf spot symptoms appear as regular circular to lanceolate lesions on plant leaves and stems.

Certain of the ethylenic imidazoles and triazoles of this invention demonstrate greater than 90% control over Botrytis fabae when applied to run-off in a liquid carrier containing 300 ppm of the candidate fungicide.

EXAMPLE C - Bean Powdery Mildew (Erysiphe polygoni) (BPM)

Bean plants (var. Dwarf Hort) are thinned to two plants per pot 24 hours prior to chemical application.

Erysiphe polygoni is cultured on bean leaves for 10 - 21 days under existing greenhouse conditions. Spores are harvested by adding deionized water containing 0.5 ml of Tween 80 per 500 ml water to a quart jar contianing excised mildew infested bean leaves. The spores are loosened from the leaf surface by shaking the jar. The resulting suspension is filtered through cheesecloth to remove plant debris and adjusted to 2-2.5 x $10^4$ spores per ml.

Bean plants are inoculated by spraying the leaves and stems with inoculum until a uniform film of inoculum is observed on the plant. Inoculated plants are maintained under existing greenhouse conditions.

Treatment comparisons are made 8 - 10 days after inoculation. Typical bean powdery mildew signs are circular white mycelial mats (fructifications) on the leaf surface.

The majority of the ethylenic imidazoles and triazoles tested gave complete control over Erysiphe polygoni.

EXAMPLE D - Grape Downy Mildew (Plasmopora viticola) (GDM)

Grape seedlings 10 - 13 cm tall are used.

Plasmopora viticola is cultured on grape leaves for 7 days at 18-24$^{o}$C in a growth room at moderate light intensity. Spores are harvested by adding deionized water and scraping the leaf surface with a camels hair brush. The spore suspension is filtered through cheesecloth to remove plant debris and adjusted to a concentration of 100 - 125,000 spores per ml.

The grape plants are inoculated by spraying the leaves with a hand held air brush until small uniform droplets of inoculum are observed on the leaves. The inoculated plants are incubated in a humid environment at 18-21°C for 48 hours prior to being placed in a growth room.

Treatment comparisons are made 7 days after inoculation. Typical grape downy mildew symptoms appear on the upper leaf surface as pale-yellow spots variable in size and form, frequently circular without a distinct line of demarcation. Under humid conditions the lower leaf surface is covered by conspicuous fungal growth.

Certain of the ethylenic imidazole and triazole compounds tested provided greater than 90% control over *Plasmopora viticola*.

EXAMPLE E - Cercospora Leafspot of Peanut (*Cercospora arachidicola*) (PC)

Peanut plants (var. Tamnut 74) are 14 days-old when treated.

*Cercospora arachidicola* is cultured on peanut-oatmeal agar (POA) in petri plates for 14 days under fluorescent lights that are 20 cm above the cultures. These petri plates are inoculated with 0.5 ml of a spore suspension made in sterile water containing a few drops of Tween 80. The spore suspension is subsequently spread over the surface of the POA plate by means of a sterile glass rod bent in the form of a hockey stick. Spores are harvested from plates by adding deionized water containing a small amount of Tween 80 to the POA plates. The agar surface is scaped with a rubber tipped glass rod. The spore suspension is filtered through cheesecloth to remove mycelial and agar fragments and then adjusted to a concentration of 75,000-100,000 spores per ml.

Treated peanut plants are inoculated by spraying the leaves with inoculum until a uniform film of inoculum is observed on the plant. Inoculated plants are incubated in a humid environment at 29-32°C for 72 hours. They are removed from the humid environment, allowed to dry, and placed under existing greenhouse conditions.

Treatment comparisons are made 10-14 days after inoculation. Typical Cercospora leafspot symptoms are brown to dark circular spots usually surrounded by a yellow halo.

Certain of the ethylenic imidazole and triazole compounds tested gave complete control of Cercospora arachidicola.

EXAMPLE F - Rice Blast (Piricularia oryzae) (RB)

Rice plants (var. Nova 66) are trimmed to a height of approximately 13 cm, 24 hours prior to chemical application. This procedure provides plants of uniform height and permits rapid inoculation of treated plants.

Piricularia oryzae is cultured on wheat dextrose agar (WDA) plates for 14 days at ambient temperature and normal room light intensity. Spores are harvested by adding deionized water containing 2 g gelatin and surface with a rubber tipped glass rod. The spore suspension is filtered through cheesecloth to remove mycelial and agar fragments and then adjusted to a concentration of 7.5-10 x $10^4$ spores/ml.

Rice plants are inoculated by spraying the leaves and stems with an air brush until a uniform film of inoculum is observed on the leaves. The inoculated plants are incubated in a humid environment (24-29°C) for 24 hours prior to being placed in a greenhouse environment.

Treatment comparisons are made 7 - 8 days after inoculation. Initial rice blast lesions appear as small brown necrotic spots on the foliage. The typical lesion is eliptical, 1 - 2 cm with a large necrotic gray center and brown margins.

Certain of the ethylenic imidizoles and triazole compounds tested showed greater than 90% control of Piricularia oryzae.

EXAMPLE G - Tomato Late Blight (Phytophthora infestans) (TLB)

Tomato (var. Rutgers) seedlings, 6 - 7.5cm tall, are fertilized with a water soluble fertilizer 4 - 5 days prior to chemical application to promote rapid succulent growth and better symptom expression.

The pathogen is grown on lima bean agar for 12 - 15 days at 16°C and the fungal growth is removed by the agitation of a rubber tipped glass rod over the surface of the agar in the presence of deionized water. The inoculum is strained through cheesecloth to remove mycelial and agar fragments and the spore concentration adjusted to 50 - 60,000 spores/ml.

The spore suspension is applied on the leafe undersurface until fine driplets are formed.

Inoculated seedlings are placed in a humid environment at 16 - 17°C for 40 - 45 hours, prior to being placed in the greenhouse at 21 - 24°C.

Treatment comparisons are made 5 - 6 days after inoculation. Initially, typical tomato late blight symptoms appear as irregular, greenish-black, water-soaked patches which enlarge and become brown, with a firm corrugated surface. Severe infection will resemble frost damage.

Certain of the ethylenic imidazole and triazole compounds tested gave greater than 90% control of Phytophthora infestans.

EXAMPLE H - Wheat Stem Rust (Puccinia graminis f. sp. tritici race 15B-2) (WSR)

Seven-day-old wheat plants (var. Wanser) are trimmed to approximately 6.4 cm 24 hours prior to chemical application to provide a uniform plant height and to facilitate uniform inoculation.

Wheat stem rust is cultured on wheat (var. Wanser) seedlings for a period of 14 days under existing greenhouse conditions.

A spore suspension of Puccinia graminis f. sp. tritici race 15B-2 is made by excising infected wheat leaves and placing the leaves into a 473 ml jar containing water and the surfactant "Tween 80" (0.5 ml/100 ml). The surfactant serves to free the rust uredio-spores from the sori and improves inoculum retention when applied to plant foliage. The resulting spore suspension is filtered through cheesecloth to remove the leaves and assorted other plant debris. The spore concentration is not adjusted, but a minimum of 2.5 x $10^4$ spores per ml are required to obtain an acceptable disease level.

Wheat plants are inoculated by applying the stem rust spore suspension until run-off. After inoculation, the plants are placed into a humid environment at approximately 20°C. A timer is used to permit 12 hours of continuous darkness followed by a minimum of 3 - 4 hours of light with an intensity of 5382 lumens/sq m (500 ft. candles). The temperature in the chamber does not exceed 29°C. At the end of the light period, the plants are allowed to dry slowly prior to being placed into a greenhouse environment.

The plants are permitted to grow under greenhouse condition for a period of 2 weeks prior to making treatment comparisons. Wheat stem rust is characterized by brick red spores in irregularly shaped sori on the leaves and stems of the wheat seedlings.

Certain of the ethylenic imidazole and triazole compounds showed complete control of Puccinia graminis.

The results of testing the compounds Examples 1 to 16 are summarized in Table III.

TABLE III

Fungicide Test Results

| Example | BH | BOT | BPM | GDM | PC | RB | TLB | WSR |
|---|---|---|---|---|---|---|---|---|
| 1 | E | C | A | E | - | - | - | E |
| 2 | A | E | A | E | - | E | E | E |
| 3 | B | C | C | E | - | C | C | E |
| 4 | E | E | E | E | E | E | E | A |
| 5 | A | E | B | C | E | C | E | A |
| 6 | B | E | E | C | - | E | C | A |
| 7 | A | E | B | E | - | E | E | A |
| 8 | A | C | A | B | B | B | C | B |
| 9 | E | B | E | E | E | E | E | E |
| 10 | C | B | C | E | E | E | E | C |
| 11 | A | E | A | E | - | E | E | A |
| 12 | A | E | A | E | - | E | E | A |
| 13 | A | E | A | B | A | A | C | A |
| 14 | A | E | A | E | A | E | E | A |
| 15 | E | C | A | E | E | E | E | E |
| 16 | E | E | A | E | E | E | E | E |

In compiling this table, the following codes are used:

BH = Barley Net Blotch (Helminthosporium teres)
BOT = Chocolate Spot of Broad Beans (Botrytis fabae)
BMP = Bean Powdery Mildew (Erysiphe polygoni)
GDM = Grape Downy Mildew (Plasmopora viticola)
PC = Peanut Cercospora (Cercospora arachidicola)
RB = Rice Blast (Piricularia oryzae)
TLB = Tomato Late Blight (Phytophthora infestans)
WSR = Wheat Stem Rust (Puccinia graminis f. sp. tritici race 15B-2)

The following disease rating scale is used for evaluating the fungicides.

A = 97-100% disease control
B = 90-96% " "
C = 70-80% " "
D = 50-60% " "
E = 50% " "

The ethylenic imidazole and triazole compounds, are useful for combating fungi by application to various locu such as the seed, the plant habitat or the foliage. For such purposes these compounds can be used in the technical or pure form as prepared, as solutions or as formulations. The compounds are usually taken up in a carrier or are formulated so as to render them suitable for subsequent dissemination as fungicides. For example, these chemical agents can be formulated as wettable powders, emulsifiable concentrates, dusts, granular formulations, aerosols, or flowable emulsion concentrates. In such formulations, the compounds are extended with a liquid or solid carrier and, when desired, suitable surfactants are incorporated.

Generally at least one of the ethylenic imidazole and triazole compounds, including of course the acid addition salts and metal salt complexes will be used in association with an agronomically acceptable carrier or diluent. By the term "agronomically acceptable carrier or diluents" is meant any substance which can be utilized to dissolve, disperse, or diffuse the chemical agent incorporated therein without impairing the effectiveness of the chemical agent and which does no permanent damage to such environment as the soil, the equipment and the agronomic crops.

It is usually desirable, particularly in the case of foliar spray formulations, to include adjuvants, such

as wetting agents, spreading agents, dispersing agents, stickers, adhesive and the like in accordance with agricultural practices. Such adjuvants commonly used in the art can be found in the John W. McCutcheon, Inc. publication "Detergents and Emulsifiers, Annual."

In general, the compounds of this invention can be dissolved in certain solvents such as acetone, methanol, ethanol, dimethylformamide, pyridine or dimethyl sulfoxide and such solutions can be extended with water. The concentrations of the solution can vary from about 1% to about 90% by weight with a preferred range being from about 5% to about 50% by weight.

For the preparation of emulsifiable concentrates, the compound can be dissolved in suitable organic solvents, or mixtures of solvents, together with an emulsifying agent which permits dispersion of the fungicide in water. The concentration of the active ingredient in emulsifiable concentrates is usually from about 10% to about 90% by weight and in flowable emulsion concentrates, this can be as high as about 75% by weight.

Wettable powders suitable for spraying, can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations is usually in the range of from about 20% to about 98% by weight preferably from about 40% to about 75% by weight. A typical wettable powder is made by blending 50 parts of 1-[2-(2,4-dichlorophenyl hex-1-enyl] imidazole, 45 parts of a synthetic precipitated hydrated silicon dioxide sold under the trademark Hi-Sil, and 5 parts of sodium lignosulfonate. In another preparation a kaolin

type (Barden) clay is used in place of the Hi-Sil in the above wettable powder, and in another such preparation 25% by weight of the Hi-Sil is replaced with a synthetic sodium silico aluminate sold under the trademark Zeolex 7.

Dusts are prepared by mixing the ethylenic imidazoles and triazoles, enantiomorphs, salts and complexes thereof with finely divided inert solids which can be organic or inorganic in nature. Materials useful for this purpose include botanical flours, silicas, silicates, carbonates and clays. One convenient method of preparing a dust is to dilute a wettable powder with a finely divided carrier. Dust concentrates containing from about 20% to about 80% by weight of the active ingredient are commonly made and are subsequently diluted to from about 1% to about 10% by weight use concentration.

The ethylenic imidazoles and triazoles, enantiomorphs, geometric isomers, salts and complexes thereof can be applied as fungicidal sprays by methods commonly employed, such as conventional high-gallonage hydraulic sprays, low-gallonage sprays, air-blast spray, aerial sprays and dusts. The dilution and rate of application will depend upon the type of equipment employed, the method of application and diseases to be controlled, but the preferred effective amount is usually from about 0.3 to about 11 kg/ha of the active ingredient.

As a seed protectant, the amount of toxicant coated on the seed is usually at a dosage rate of from about 3 to about 570 g per 45 kg of seed. As a soil fungicide the chemical can be incorporated in the soil or applied to the surface usually at a rate of from about 0.3 to about 11 kg/ha.

Fungicides which can be combined with the fungicides of this invention include those listed on pages 47 and 48 of U.K. Patent Specification No. 1,530,172 cited earlier.

The ethylenic imidazoles and triazoles, enantiomorphs, acid addition salts and metal salt complexes of this invention can be advantageously employed in various ways. Since these compounds possess broad spectrum fungicidal activity, they can be employed in the storage of cereal grain. These complexes can also be employed as fungicides in turf, fruit orchards, vegetables and golf course applications.

Claims:-

1. An N-substituted 1-H-imidazole, 1-H-1,2,4-triazole or 4-H-1,2,4-triazole, an agronomically acceptable acid addition salt thereof or an agronomically acceptable metal salt complex thereof, characterized in that the imidazole or triazole is of the formula:

$$ZR^1C = CQR^2$$

wherein:

Z is phenyl or naphthyl each being optionally substituted with up to three of the same or different substituents selected from halogen, nitro, trihalomethyl, cyano, $(C_1-C_6)$alkyl, $(C_1-C_6)$alkoxy, $(C_1-C_6)$alkylthio, $(C_1-C_6)$-alkylsulfinyl, $(C_1-C_6)$alkylsulfonyl and $(C_2-C_6)$alkenyl;

$R^1$ is hydrogen, $(C_1-C_{12})$alkyl, $(C_3-C_8)$cycloalkyl, $(C_2-C_8)$alkenyl, $(C_5-C_8)$cycloalkenyl, $(C_2-C_8)$-alkynyl, phenyl, naphthyl each of the last groups being optionally substituted with up to three of the same or different substituents selected from halogen, nitro, trihalomethyl, cyano, $(C_1-C_6)$alkyl, $(C_2-C_6)$alkenyl, $(C_2-C_6)$-alkynyl, $(C_1-C_6)$alkoxy, $(C_1-C_6)$alkylsulfinyl and $(C_1-C_6)$alkylsulfonyl;

$R^2$ is hydrogen, phenyl optionally substituted with up to three of the same or different substituents selected from nitro, trihalomethyl,

cyano, $(C_1-C_6)$alkyl, $(C_2-C_6)$alkenyl, $(C_2-C_6)$alkynyl, $(C_1-C_6)$alkoxy, $(C_1-C_6)$-alkylsulfinyl and $(C_1-C_6)$alkylsulfonyl; and

Q is 1-imidazolyl, or 1- or 4-(1,2,4-triazolyl).

2. A compound salt or metal salt complex as claimed in Claim 1, wherein Z is phenyl optionally substituted with up to two substituents selected from chlorine, bromine, $(C_1-C_4)$alkyl, nitro, trifluoromethyl, cyano and $(C_1-C_4)$alkoxy; $R^1$ is hydrogen, $(C_1-C_8)$alkyl, phenyl optionally substituted with up to two of the same or different substituents selected from chlorine, bromine, nitro, trifluoromethyl, cyano, $(C_1-C_4)$alkyl and $(C_1-C_4)$alkoxy; and Q is 1-imidazolyl, 1- or 4-(1,2,4-triazolyl).

3. A compound, salt or metal salt complex as claimed in Claim 1, wherein Z is phenyl optionally substituted with up to two substituents selected from halo and $(C_1-C_4)$alkyl, $R^1$ is hydrogen, $(C_1-C_6)$alkyl or phenyl optionally substituted with up to two of the same or different substituents selected from $(C_1-C_4)$alkyl or halogen, $R^2$ is hydrogen or phenyl substituted with up to two of the same or different halogen atoms and Q is 1-imidazolyl or 1-(1,2,4-triazolyl).

4. A compound salt or metal salt complex as claimed in Claim 1, wherein Z is phenyl or phenyl substituted with up to two substituents selected from chlorine, bromine and $(C_1-C_4)$alkyl; $R^1$ is hydrogen, $(C_1-C_6)$alkyl, phenyl, or phenyl substituted with up to two substituents selected from chloro, bromo and $(C_1-C_4)$-alkyl; $R^2$ is hydrogen, phenyl, or phenyl substituted with up to two substituents selected from chlorine, bromine and $(C_1-C_4)$alkyl; and Q is 1-imidazolyl or 1-(1,2,4-triazolyl).

5. A compound,salt or metal salt complex as claimed in Claim 4, wherein Z is phenyl, 4-chlorophenyl, 2-chlorophenyl or 2,4-dichlorophenyl; $R^1$ is hydrogen, $(C_1-C_4)$alkyl, phenyl, 2-chlorophenyl, or 4-chlorophenyl and $R^2$ is hydrogen, phenyl, or 2-chlorophenyl.

6. A compound, salt or metal salt complex as claimed in Claim 1, wherein (A) Q is 1-imidazolyl, Z is 2-chlorophenyl, $R^1$ is H and $R^2$ is 2-chlorophenyl, (B) Q is 1-imidazolyl, $R^2$ is hydrogen and (a) Z is 2,4-dichlorophenyl and $R^1$ is n-butyl, (b) Z and $R^1$ are each 4-chlorophenyl, and (c) Z and $R^1$ are each phenyl, (d) Z is 4-chlorophenyl and $R^1$ is 2-chlorophenyl, (e) Z and $R^1$ is each 4-ethylphenyl or (C) Q is 1-(1,2,4-triazolyl), $R^2$ is hydrogen and (a) Z is 4-chlorophenyl and $R^1$ is 2- or 4-chlorophenyl, (b) Z is 2,4-dichlorophenyl and $R^1$ is n-butyl or (c) Z and $R^1$ are each 4-ethylphenyl.

7. A salt as claimed in Claim 6 being an acid addition salt of hydrochloric or nitric acid.

8. A fungicidal composition containing as active ingredient, at least one compound, salt or metal salt complex as claimed in any preceding claim plus an agronomically acceptable carrier or diluent for the active ingredient.

9. A method of combating phytopathogenic fungi which comprises applying to the plant, the plant habitat or plant seeds at least one compound as claimed in any one of Claims 1-7.

Claims:- AUSTRIA

1. A fungicidal composition containing (A), as active ingredient, at least one N-substituted 1-H-imidazole, 1-H-1,2,4-triazole or 4-H-1,2,4-triazole, an agronomically acceptable acid addition salt thereof or an agronomically acceptable metal salt complex thereof and (B) an agronomically acceptable carrier or diluent for the active ingredient, characterized in that the imidazole or triazole is of the formula:

$$ZR^1C = CQR^2$$

wherein:

Z is phenyl or naphthyl each being optionally substituted with up to three of the same or different substituents selected from halogen, nitro, trihalomethyl, cyano, $(C_1-C_6)$alkyl, $(C_1-C_6)$alkoxy, $(C_1-C_6)$alkylthio, $(C_1-C_6)$-alkylsulfinyl, $(C_1-C_6)$alkylsulfonyl and $(C_2-C_6)$alkenyl;

$R^1$ is hydrogen, $(C_1-C_{12})$alkyl, $(C_3-C_8)$cycloalkyl, $(C_2-C_8)$alkenyl, $(C_5-C_8)$cycloalkenyl, $(C_2-C_8)$-alkynyl, phenyl, naphthyl each of the last groups being optionally substituted with up to three of the same or different substituents selected from halogen, nitro, trihalomethyl, cyano, $(C_1-C_6)$alkyl, $(C_2-C_6)$alkenyl, $(C_2-C_6)$-alkynyl, $(C_1-C_6)$alkoxy, $(C_1-C_6)$alkylsulfinyl and $(C_1-C_6)$alkylsulfonyl;

$R^2$ is hydrogen, phenyl optionally substituted with up to three of the same or different substituents selected from nitro, trihalomethyl,

cyano, $(C_1-C_6)$alkyl, $(C_2-C_6)$alkenyl, $(C_2-C_6)$alkynyl, $(C_1-C_6)$alkoxy, $(C_1-C_6)$-alkylsulfinyl and $(C_1-C_6)$alkylsulfonyl; and

Q is 1-imidazolyl, or 1- or 4-(1,2,4-triazolyl).

2. A composition as claimed in Claim 1, wherein Z is phenyl optionally substituted with up to two substituents selected from chlorine, bromine, $(C_1-C_4)$-alkyl, nitro, trifluoromethyl, cyano and $(C_1-C_4)$alkoxy; $R^1$ is hydrogen, $(C_1-C_8)$alkyl, phenyl optionally substituted with up to two of the same or different substituents selected from chlorine, bromine, nitro, trifluoromethyl, cyano, $(C_1-C_4)$alkyl and $(C_1-C_4)$alkoxy; and Q is 1-imidazolyl, 1- or 4-(1,2,4-triazolyl).

3. A composition as claimed in Claim 1, wherein Z is phenyl optionally substituted with up to two substituents selected from halo and $(C_1-C_4)$alkyl, $R^1$ is hydrogen, $(C_1-C_6)$alkyl or phenyl optionally substituted with up to two of the same or different substituents selected from $(C_1-C_4)$alkyl or halogen, $R^2$ is hydrogen or phenyl substituted with up to two of the same or different halogen atoms and Q is 1-imidazolyl or 1-(1,2,4-triazolyl).

4. A composition as claimed in Claim 1, wherein Z is phenyl or phenyl substituted with up to two substituents selected from chlorine, bromine and $(C_1-C_4)$alkyl; $R^1$ is hydrogen, $(C_1-C_6)$alkyl, phenyl, or phenyl substituted with up to two substituents selected from chloro, bromo and $(C_1-C_4)$alkyl; $R^2$ is hydrogen, phenyl, or phenyl substituted with up to two substituents selected from chlorine, bromine and $(C_1-C_4)$alkyl; and Q is 1-imidazolyl or 1-(1,2,4-triazolyl).

5. A composition as claimed in Claim 4, wherein Z is phenyl, 4-chlorophenyl, 2-chlorophenyl or 2,4-dichlorophenyl; $R^1$ is hydrogen, $(C_1-C_4)$alkyl, phenyl, 2-chlorophenyl, or 4-chlorophenyl and $R^2$ is hydrogen, phenyl, or 2-chlorophenyl.

6. A composition as claimed in Claim 1, wherein (A) Q is 1-imidazolyl, Z is 2-chlorophenyl, $R^1$ is H and $R^2$ is 2-chlorophenyl, (B) Q is 1-imidazolyl, $R^2$ is hydrogen and (a) Z is 2,4-dichlorophenyl and $R^1$ is n-butyl, (b) Z and $R^1$ are each 4-chlorophenyl, and (c) Z and $R^1$ are each phenyl, (d) Z is 4-chlorophenyl and $R^1$ is 2-chlorophenyl, (e) Z and $R^1$ is each 4-ethylphenyl or (C) Q is 1-(1,2,4-triazolyl), $R^2$ is hydrogen and (a) Z is 4-chlorophenyl and $R^1$ is 2- or 4-chlorophenyl, (b) Z is 2,4-dichlorophenyl and $R^1$ is n-butyl or (c) Z and $R^1$ are each 4-ethylphenyl.

7. A composition as claimed in Claim 6 being an acid addition salt of hydrochloric or nitric acid.

8. A method of combating phytopathogenic fungi which comprises applying to the plant, the plant habitat or plant seeds a composition as claimed in any one of Claims 1-7.